# EUROPÄISCHE PATENTANMELDUNG

(11) **EP 4 632 157 A1**
(43) Veröffentlichungstag der Anmeldung: **15.10.2025**
(21) Anmeldenummer: 25170086.0
(22) Anmeldetag: 11.04.2025
(51) Int. Cl.: E03B 7/04, E03B 7/09

(54) **LEITUNGSSYSTEM MIT ZIRKULATIONSLEITUNG**

(30) Priorität: 12.04.2024 DE 202024101802 U
(71) Anmelder: Lauer AG, 4051 Basel (CH)
(72) Erfinder: Lauer, Hans Günter, 4125 Riehen (CH)
(74) Vertreter: Braunpat AG

(57) **Zusammenfassung**

Die vorliegende Erfindung betrifft ein Leitungssystem (100), insbesondere ein Frisch- oder Reinwasser-Rohrleitungssystem, mit
a) einer Hauptleitung (10), die von einem Medium durchströmbar ist, und mit
b) zumindest einer davon abgezweigten, einen Bypass bildenden Nebenleitung (20), an die wenigstens eine Entnahmestelle (30) anschliessbar ist,
- wobei die Hauptleitung (10) mindestens einen Bereich (60) mit vergrössertem Innendurchmesser und mindestens einen Bereich (70) mit verkleinertem Innendurchmesser aufweist,
- wobei jeweils der zuströmseitige Leitungsabschnitt (40, 41) einer Nebenleitung einem Bereich (60) der Hauptleitung mit vergrössertem Innendurchmesser zugeordnet ist,
- wobei jeweils der abströmseitige Leitungsabschnitt (50, 51) einer Nebenleitung einem Bereich der Hauptleitung (70) mit verkleinertem Innendurchmesser zugeordnet ist,

**dadurch gekennzeichnet, dass**
zumindest einer der zuströmseitigen oder abströmseitigen Leitungsabschnitte (40, 41, 50, 51) der Nebenleitung(en) mit der Hauptleitung (10) in einem schiefen Winkel α zur Längsachse der Hauptleitung in Hauptfliessrichtung (1) verbunden ist.

## Beschreibung

### Technisches Gebiet der Erfindung

Die Erfindung betrifft ein Leitungssystem, insbesondere ein Frisch- oder Reinwasser-Rohrleitungssystem für die pharmazeutische Wasserversorgung wie sie beispielsweise bei der Dialysewasserversorgung benötigt wird. Das erfindungsgemässe Leitungssystem ist möglichst kontaminationsfrei und weist geringstmögliche Ablagerungen bis zur Entnahmestelle, dem sog. "Point of Use", auf. Zudem kann das erfindungsgemässe Leitungssystem die in der pharmazeutischen Wasserversorgung geforderten Fliessgeschwindigkeiten des Wassers insbesondere im Bereich von 0,45 bis 1,75 m/s, bevorzugt im Bereich von 0,60 bis 1,5 m/s, erreichen, was mit bisherigen Systemen nicht, oder sehr schwer, erreicht wurde. Somit ist das Wasser dann auch für Injektionszwecke geeignet.

Das erfindungsgemässe Leitungssystem ist auch für jede andere Wasserversorgung einsetzbar wie z.B. für die Trinkwasser-Versorgung oder die Wasserversorgung für weitere Bereiche, die eine hohe Wasserqualität erfordern, wie beispielsweise in der Chipfabrikation.

### Stand der Technik

Um beispielsweise qualitativ hochwertiges Reinwasser an verschiedenen Entnahmestellen bereitstellen zu können, ist es bereits bekannt, ein Rohrleitungsnetz mit einer Haupt-Ringleitung vorzusehen, die ständig von einem Wasserstrom durchspült wird. Durch diesen kontinuierlichen Flüssigkeitsstrom wird solchen Verunreinigungen entgegengewirkt, die sich bei einem Stehen des Wassers im Leitungssystem bilden oder ansammeln könnten. Aus demselben Grund sind die Entnahmestellen mit der Hauptleitung jeweils über eine Nebenleitung verbunden, die praktisch als Bypass ausgebildet ist und einen zu- und abströmseitigen Leitungsabschnitt aufweist.

Um auch in diesen zu einer Entnahmestelle führenden Nebenleitungen das Wasser selbst bei diskontinuierlicher Entnahme in einem kontinuierlichen Flüssigkeitsstrom durchfliessen zu lassen, sind in die Haupt-Ringleitung des vorbekannten Rohrleitungsnetzes im Bereich jeder Entnahmestelle Rohrabschnitte zwischengeschaltet, in denen sich die Ringleitung konisch oder düsenförmig verengt, um sich anschliessend wieder zu erweitern. Dabei ist der zuströmseitige Leitungsabschnitt jeder Nebenleitung ausserhalb der ihr zugeordneten Rohrverengung angeordnet, während der abströmseitige Leitungsabschnitt in dem Bereich der Rohrverengung mündet, der den geringsten Innendurchmesser hat.

Im Bereich dieser Rohrverengung wird die Fliessgeschwindigkeit des in der Haupt-Ringleitung fliessenden Flüssigkeitsstromes erhöht und gleichzeitig der statische Druck in diesem Bereich reduziert. Der von verkleinerten Innendurchmessern der Rohrleitung erzeugte Erniedrigung des statischen Drucks bewirkt den erwünschten kontinuierlichen Flüssigkeitsstrom auch in der Nebenleitung.

Um sicherzustellen, dass an den Entnahmestellen ein genügend hoher Wasserdruck herrscht, wurde das in der DE 43 41 456 A beschriebene Leitungssystem bereits mit Rohrerweiterungen in der Hauptleitung versehen. Dank den Rohrerweiterungen wird der statische Druck erhöht, was zu einer Erhöhung der Fliessgeschwindigkeit in den Nebenleitungen führt.

Die Aufgabe der vorliegenden Erfindung ist es, ein verbessertes Leitungssystem vorzuschlagen, und insbesondere Fliessgeschwindigkeiten von 0,45 bis 1,75 m/s bis zum "Point of Use" zu ermöglichen.

### Zusammenfassung der Erfindung

Diese Aufgabe wird insbesondere gelöst durch ein Leitungssystem, insbesondere ein Frisch- oder Reinwasser-Rohrleitungssystem, mit
a) einer Hauptleitung, die von einem Medium durchströmbar ist, und mit
b) zumindest einer davon abgezweigten, einen Bypass bildenden Nebenleitung, an die wenigstens eine Entnahmestelle anschliessbar ist,
   - wobei die Hauptleitung mindestens einen Bereich mit vergrössertem Innendurchmesser und mindestens einen Bereich mit verkleinertem Innendurchmesser aufweist,
   - wobei jeweils der zuströmseitige Leitungsabschnitt einer Nebenleitung einem Bereich der Hauptleitung mit vergrössertem Innendurchmesser zugeordnet ist,
   - wobei jeweils der abströmseitige Leitungsabschnitt einer Nebenleitung einem Bereich der Hauptleitung mit verkleinertem Innendurchmesser zugeordnet ist, wobei
   - zumindest einer der zuströmseitigen oder abströmseitigen Leitungsabschnitte der Nebenleitung(en) mit der Hauptleitung in einem schiefen Winkel α zur Längsachse der Hauptleitung in Hauptfliessrichtung verbunden ist.

Das erfindungsgemässe Leitungssystem kann mehrere dieser Nebenleitungen umfassen.

Durch die Verbindung des zuströmseitigen Leitungsabschnitts der Nebenleitung(en) mit der Hauptleitung in einem schiefen Winkel α zur Längsachse der Hauptleitung in Hauptfliessrichtung wird der Druck auf der Zuströmseite noch weiter erhöht. Die schiefe Anordnung des zuströmseitigen Leitungsabschnitts bewirkt, dass ein Teil des dynamischen Drucks des Mediums in die Nebenleitung abgegeben wird. Mit der Verbindung des abströmseitigen Leitungsabschnitts der Nebenleitung(en) mit der Hauptleitung in einem schiefen Winkel α zur Längsachse der Hauptleitung in Hauptfliessrichtung wird eine weitere Erniedrigung des Drucks auf der Abströmseite erreicht.

Das erfindungsgemässe Leitungssystem zeichnet sich also dadurch aus, dass sich durch die schief angeordneten zuström- und/oder abströmseitigen Leitungsabschnitte der mindestens einen Nebenleitung der Druck verstärkt, und damit die Fliessgeschwindigkeit in der Nebenleitung erhöht wird, und so die in der pharmazeutischen Wasserversorgung wie beispielsweise der Dialysewasserversorgung geforderten Fliessgeschwindigkeiten von mindestens 0,45 m/s, insbesondere Fliessgeschwindigkeiten im Bereich von 0,60 bis 1,5 m/s, erreicht werden kann.

In einer bevorzugten Ausführungsform des Leitungssystems gemäss der vorliegenden Erfindung ist der einer Nebenleitung zugeordnete Bereich der Hauptleitung mit verkleinertem Innendurchmesser dem der gleichen Nebenleitung zugeordneten Bereich der Hauptleitung mit vergrössertem Innendurchmesser in Hauptfliessrichtung der Hauptleitung vorgeschaltet. Dies führt zu einer weiteren Druckerhöhung in der Nebenleitung.

In einer bevorzugten Ausführungsform des Leitungssystems gemäss der vorliegenden Erfindung ist der zuströmseitige Leitungsabschnitt der Nebenleitung mit der Hauptleitung in einem stumpfen Winkel α, vorteilhafterweise in einem Winkel von mindestens 135°, zur Längsachse der Hauptleitung in Hauptfliessrichtung verbunden. Dadurch wird der Druck im zuströmseitigen Leitungsabschnitt der Nebenleitung erhöht, wenn das Medium in Hauptfliessrichtung fliesst.

In einer weiteren bevorzugten Ausführungsform des Leitungssystems gemäss der vorliegenden Erfindung ist der abströmseitige Leitungsabschnitt der Nebenleitung mit der Hauptleitung in einem spitzen Winkel α, vorteilhafterweise in einem Winkel von maximal 45°, zur Längsachse der Hauptleitung in Hauptfliessrichtung verbunden. Dadurch wird der Druck im abströmseitigen Leitungsabschnitt der Nebenleitung erniedrigt, wenn das Medium in Hauptfliessrichtung fliesst.

Jede der Nebenleitungen in dem erfindungsgemässen Leitungssystem kann mehrere zuströmseitige und/oder mehrere abströmseitige Leitungsabschnitte umfassen, welche in einem schiefen Winkel α zur Längsachse der Hauptleitung in Hauptfliessrichtung angeordnet sind. So kann beispielsweise jede Nebenleitung in dem erfindungsgemässen Leitungssystem zwei zuströmseitige und zwei abströmseitige Leitungsabschnitte umfassen.

In einer weiteren bevorzugten Ausführungsform des Leitungssystems gemäss der vorliegenden Erfindung weist die mindestens eine Nebenleitung einen ersten zuströmseitigen Leitungsabschnitt und einen zweiten zuströmseitigen Leitungsabschnitt auf. Hierbei sind sowohl der erste zuströmseitige Leitungsabschnitt als auch der zweite zuströmseitige Leitungsabschnitt der mindestens einen Nebenleitung demselben Bereich der Hauptleitung mit vergrössertem Innendurchmesser zugeordnet. In einer weiteren bevorzugten Ausführungsform ist hierbei der zweite zuströmseitige Leitungsabschnitt der Nebenleitung mit der Hauptleitung in einem spitzen Winkel α zur Längsachse der Hauptleitung in Hauptfliessrichtung verbunden. Dadurch wird der Druck im zuströmseitigen Leitungsabschnitt der Nebenleitung erhöht, egal ob das Medium in Hauptfliessrichtung oder in Gegenfliessrichtung fliesst. Dies hat den Vorteil, dass der Druck im zuströmseitigen Leitungsabschnitt der Nebenleitung(en) erhöht wird, egal ob das Medium in Hauptfliessrichtung oder in Gegenfliessrichtung fliesst.

In einer weiteren bevorzugten Ausführungsform des Leitungssystems gemäss der vorliegenden Erfindung weist die Nebenleitung einen ersten abströmseitigen Leitungsabschnitt und einen zweiten abströmseitigen Leitungsabschnitt auf. Hierbei ist bevorzugt der erste abströmseitige Leitungsabschnitt als auch der zweite abströmseitige Leitungsabschnitt der Nebenleitung demselben Bereich der Hauptleitung mit verkleinertem Innendurchmesser zugeordnet. In einer weiteren bevorzugten Ausführungsform ist hierbei der zweite abströmseitige Leitungsabschnitt der Nebenleitung mit der Hauptleitung in einem spitzen Winkel α, vorteilhafterweise in einem Winkel von maximal 45°, zur Längsachse der Hauptleitung in Hauptfliessrichtung verbunden. Dies hat den Vorteil, dass der Druck im abströmseitigen Leitungsabschnitt der Nebenleitung(en) erniedrigt wird, egal ob das Medium in Hauptfliessrichtung oder in Gegenfliessrichtung fliesst.

In einer weiteren bevorzugten Ausführungsform des Leitungssystems gemäss der vorliegenden Erfindung sind alle zuströmseitigen Leitungsabschnitte und alle abströmseitigen Leitungsabschnitte aller Nebenleitungen mit der Hauptleitung in einem schiefen Winkel α zur Längsachse der Hauptleitung verbunden. Dadurch kann der Druck in allen zuströmseitigen Leitungsabschnitten erhöht und in allen abströmseitigen Leitungsabschnitten erniedrigt werden, egal ob das Medium in Hauptfliessrichtung oder in Gegenfliessrichtung fliesst.

Bevorzugt sind die Bereiche der Hauptleitung mit verändertem Innendurchmesser symmetrisch ausgebildet, was ihre Herstellung vereinfacht und billiger macht. Die Bereiche mit verändertem Innendurchmesser können sowohl benachbart zueinander oder beabstandet zueinander in der Hauptleitung angeordnet sein.

In einer weiteren bevorzugten Ausführungsform des Leitungssystems gemäss der vorliegenden Erfindung hat der Bereich der Hauptleitung mit vergrössertem Innendurchmesser zwei, vorzugsweise etwa gleich grosse, Erweiterungsabschnitte, die jeweils trichter- oder kegelstumpfförmig ausgebildet sind und sich zu ihren einander abgewandten Enden hin verjüngen.

In einer weiteren bevorzugten Ausführungsform des Leitungssystems gemäss der vorliegenden Erfindung hat der Bereich der Hauptleitung mit verkleinertem Innendurchmesser zwei, vorzugsweise etwa gleich grosse, Verengungsabschnitte, die jeweils trichter- oder kegelstumpfförmig ausgebildet sind und sich zu ihren einander zugewandten Enden hin verjüngen.

Das erfindungsgemässe Leitungssystem kann sowohl in einer Hauptfliessrichtung der Hauptleitung betrieben werden als auch in ihrer Gegenfliessrichtung. Durch diese Fliessrichtungsumkehr können Ablagerungen, welche in normaler Fliessrichtung, d.h. Hauptfliessrichtung, vorkommen, besser abgespült werden. Um diesen Effekt noch zu verstärken, kann das Wasser auch in Druckstössen, vorzugsweise in Gegenfliessrichtung, durch die Hauptleitung und damit auch die mindestens eine Nebenleitung gepumpt werden.

Die vorliegende Erfindung umfasst auch jede beliebige Kombination der Merkmale der bevorzugten Ausführungsformen, auch wenn diese Kombinationen nicht einzeln offenbart werden.

Weitere Einzelheiten der Erfindung gehen aus der nun folgenden nicht-limitierenden Beschreibung der bevorzugten Ausführungsformen der Erfindung hervor, welche in den beigelegten Zeichnungen dargestellt sind. Aus dieser Beschreibung lassen sich auch Anregungen und Vorschläge entnehmen, wie der Erfindungsgegenstand im Rahmen des Beanspruchten abgeändert oder auch weiterentwickelt werden könnte.

### Kurzbeschreibung der Zeichnungen

Fig. 1 zeigt eine erste bevorzugte Ausführungsform des erfindungsgemässen Leitungssystems, in dem der zuströmseitige Leitungsabschnitt der Nebenleitung mit der Hauptleitung in einem stumpfen Winkel α zur Längsachse der Hauptleitung in Hauptfliessrichtung verbunden ist.
Fig. 2 zeigt eine zweite bevorzugte Ausführungsform des erfindungsgemässen Leitungssystems, in dem der abströmseitige Leitungsabschnitt der Nebenleitung mit der Hauptleitung in einem spitzen Winkel α zur Längsachse der Hauptleitung in Hauptfliessrichtung verbunden ist.
Fig. 3 zeigt eine dritte bevorzugte Ausführungsform des erfindungsgemässen Leitungssystems, in dem die Nebenleitung zwei zuströmseitige Leitungsabschnitte aufweist.
Fig. 4 zeigt eine vierte bevorzugte Ausführungsform des erfindungsgemässen Leitungssystems, in dem die Nebenleitung zwei abströmseitige Leitungsabschnitte aufweist.
Fig. 5 zeigt eine fünfte bevorzugte Ausführungsform des erfindungsgemässen Leitungssystems, in dem die Nebenleitung zwei zuströmseitige Leitungsabschnitte und zwei abströmseitige Leitungsabschnitte aufweist.

### Bevorzugte Ausführungsformen der Erfindung

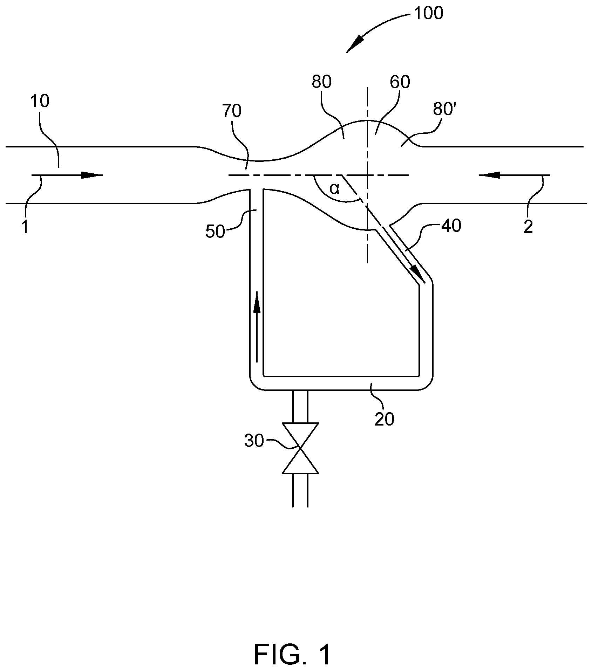

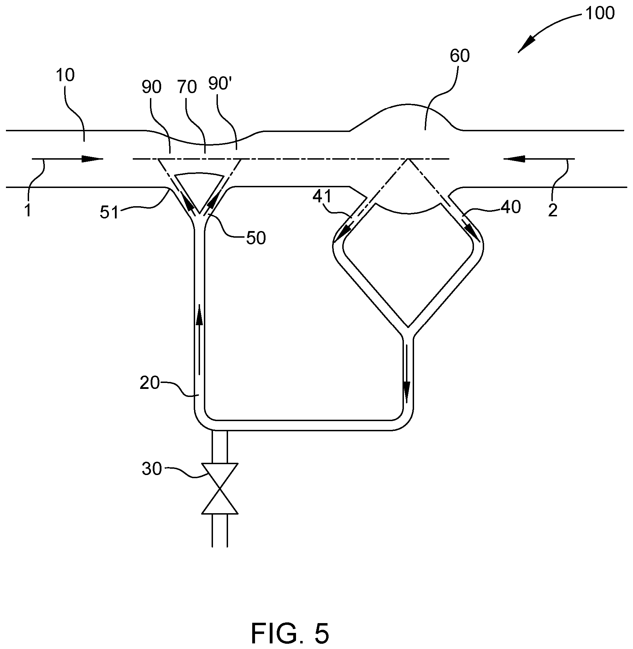

In den Fig. 1-5 ist jeweils eine bevorzugte Ausführungsform eines erfindungsgemässen Leitungssystems 100 gezeigt, welches eine Hauptleitung 10 und eine Nebenleitung 20 mit einer Entnahmestelle 30 umfasst. Selbstverständlich kann das erfindungsgemässe Leitungssystem 100 auch mehrere Nebenleitungen (20) umfassen. Hierbei weist die Hauptleitung jeweils einen Bereich 70 mit verkleinertem Innendurchmesser und einen Bereich 60 mit vergrössertem Innendurchmesser auf, wobei der Bereich 70 mit verkleinertem Innendurchmesser dem Bereich 60 mit vergrössertem Innendurchmesser in Hauptfliessrichtung 1 vorgeschaltet ist. Bevorzugt sind diese Bereiche 60 und 70 jeweils symmetrisch ausgebildet und bestehen aus annähernd gleich grossen und gleichförmigen Erweiterungsabschnitten 80 und 80' bzw. Verengungsabschnitten 90 und 90'. Die Erweiterungsabschnitten 80 und 80' sind bevorzugt jeweils trichter- oder kegelstumpfförmig ausgebildet und verjüngen sich zu ihren einander abgewandten Enden hin. Die Verengungsabschnitte 90 und 90' sind jeweils trichter- oder kegelstumpfförmig ausgebildet und verjüngen sich zu ihren einander zugewandten Enden hin. In den Ausführungsformen gemäss den Fig. 1-4 sind die Bereiche mit verändertem Innendurchmesser benachbart zueinander, während sie in der Ausführungsform gemäss Fig. 5 beabstandet zueinander sind. Die Hauptfliessrichtung 1 und die Gegenfliessrichtung 2 sind mit entsprechenden Pfeilen symbolisiert. In den Fig. 1-5 ist die Fliessrichtung des Wassers in der mindestens einen Nebenleitung 20 bzw. ihren zuströmseitigen Leitungsabschnitten 40 und 41 und ihren abströmseitigen Leitungsabschnitten 50 und 51 durch entsprechende Pfeilspitzen symbolisiert.

In der in Fig. 1 gezeigten bevorzugten Ausführungsform des erfindungsgemässen Leitungssystem 100 ist der zuströmseitige Leitungsabschnitt 40 der Nebenleitung mit einem Bereich 60 der Hauptleitung 10 mit vergrössertem Innendurchmesser in einem stumpfen Winkel α zur Längsachse der Hauptleitung in Hauptfliessrichtung 1 verbunden, während der abströmseitige Leitungsabschnitt 50 der Nebenleitung mit einem Bereich 70 der Hauptleitung 10 mit verkleinertem Innendurchmesser in einem 90° Winkel verbunden ist.

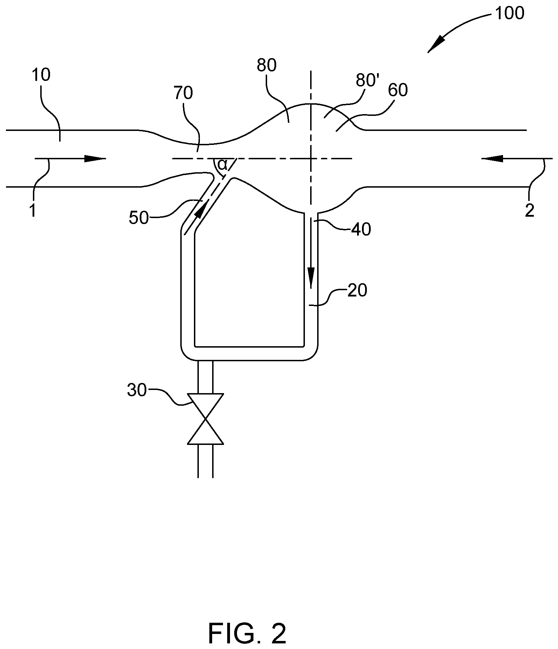

In der in Fig. 2 gezeigten Ausführungsform des erfindungsgemässen Leitungssystem 100 ist der abströmseitige Leitungsabschnitt 50 der Nebenleitung mit einem Bereich 70 der Hauptleitung 10 mit verkleinertem Innendurchmesser in einem spitzen Winkel α zur Längsachse der Hauptleitung in Hauptfliessrichtung 1 verbunden, während der zuströmseitige Leitungsabschnitt 40 der Nebenleitung mit einem Bereich 60 der Hauptleitung 10 mit vergrössertem Innendurchmesser in einem 90° Winkel verbunden ist.

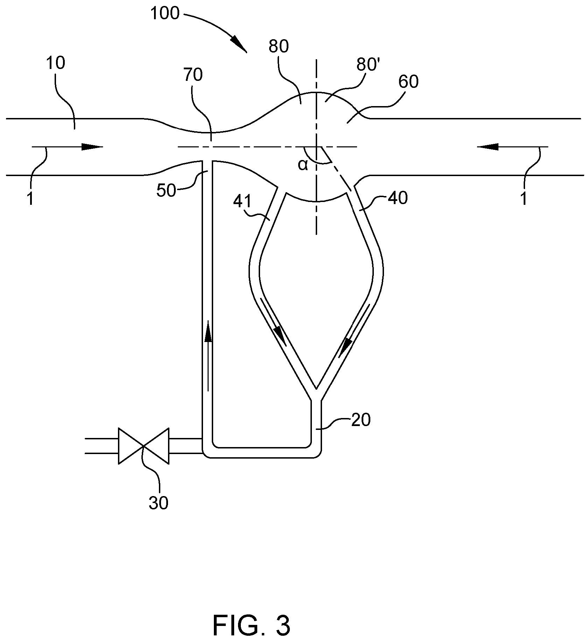

Die bevorzugte Ausführungsform gemäss Fig. 3 ist eine Abwandlung der in Fig. 1 gezeigten Ausführungsform des erfindungsgemässen Leitungssystem 100, wobei die Nebenleitung 20 zwei zuströmseitige Leitungsabschnitte 40 und 41 aufweist. Hierbei ist der erste zuströmseitige Leitungsabschnitt 40 der Nebenleitung mit der Hauptleitung 10 in einem stumpfen Winkel α und der zweite zuströmseitige Leitungsabschnitt 41 der Nebenleitung mit der Hauptleitung 10 in einem spitzen Winkel α verbunden, wobei der Winkel jeweils zur Längsachse der Hauptleitung in Hauptfliessrichtung 1 gebildet wird. Die Ausführungsform der Fig. 3 hat den Vorteil, dass der Druck im zuströmseitigen Leitungsabschnitt der Nebenleitung(en) 20 erhöht wird, egal ob das Medium in Hauptfliessrichtung oder in Gegenfliessrichtung fliesst. In Hauptfliessrichtung wird der Druck im Abschnitt 40 durch den dynamischen Druck des fliessenden Mediums erhöht. In Gegenfliessrichtung wird der Druck im Abschnitt 41 durch den dynamischen Druck des fliessenden Mediums erhöht.

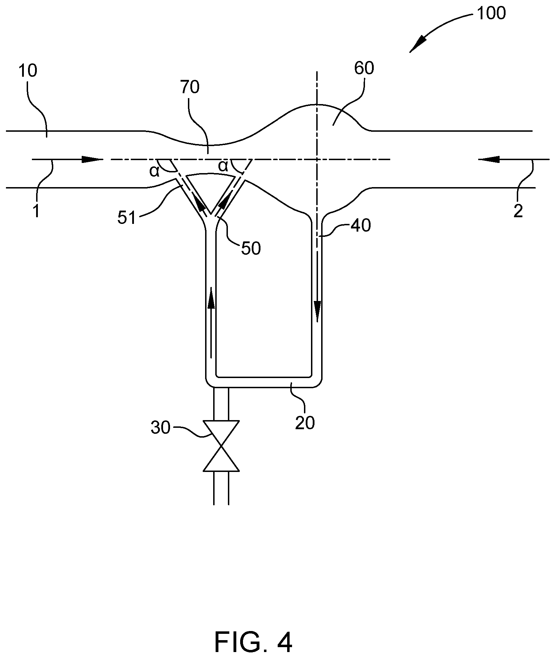

Die bevorzugte Ausführungsform gemäss Fig. 4 ist eine Abwandlung der in Fig. 2 gezeigten Ausführungsform des erfindungsgemässen Leitungssystem 100, wobei die Nebenleitung 20 zwei abströmseitige Leitungsabschnitte 50 und 51 aufweist. Hierbei ist der erste abströmseitige Leitungsabschnitt 50 der Nebenleitung mit der Hauptleitung 10 in einem spitzen Winkel α und der zweite zuströmseitige Leitungsabschnitt 51 der Nebenleitung mit der Hauptleitung 10 in einem stumpfen Winkel α, verbunden, wobei der Winkel jeweils zur Längsachse der Hauptleitung in Hauptfliessrichtung 1 gebildet wird. Aufgrund der herrschenden Druckverhältnisse bei Betrieb des erfindungsgemässen Leitungssystems 100 wird der erste abströmseitige Leitungsabschnitt 50 der Nebenleitung in Hauptfliessrichtung 1 durchströmt, während in Gegenfliessrichtung 2 der zweite zuströmseitige Leitungsabschnitt 51 der Nebenleitung durchströmt wird. Die Ausführungsform der Fig. 4 hat den Vorteil, dass der Druck im anströmseitigen Leitungsabschnitt der Nebenleitung(en) 20 erniederigt wird, egal ob das Medium in Hauptfliessrichtung oder in Gegenfliessrichtung fliesst. In Hauptfliessrichtung wird der Druck im Abschnitt 50 durch den dynamischen Druck des fliessenden Mediums erniedrigt. In Gegenfliessrichtung wird der Druck im Abschnitt 51 durch den dynamischen Druck des fliessenden Mediums erniedrigt.

In den in Fig. 1-4 gezeigten Ausführungsformen sind der Bereich 60 der Hauptleitung mit vergrössertem Innendurchmesser und der Bereich 70 der Hauptleitung mit verkleinertem Innendurchmesser benachbart zueinander angeordnet. Sie können jedoch auch wie in Figur 5 gezeigt beabstandet zueinander angeordnet sein.

In der in Fig. 5 gezeigten bevorzugten Ausführungsform des erfindungsgemässen Leitungssystem 100 sind alle zuströmseitigen Leitungsabschnitte 40 und 41, sowie alle abströmseitigen Leitungsabschnitte 50 und 51 der Nebenleitung 20 in schiefem Winkel mit der Hauptleitung 10 verbunden. Hierbei ist der erste zuströmseitige Leitungsabschnitt 40 und der zweite abströmseitige Leitungsabschnitt 51 der Nebenleitung mit der Hauptleitung 10 in einem stumpfen Winkel α und der erste abströmseitige Leitungsabschnitt 50 und der zweite zuströmseitige Leitungsabschnitt 41 der Nebenleitung mit der Hauptleitung 10 in einem spitzen Winkel α verbunden, wobei der Winkel jeweils zur Längsachse der Hauptleitung in Hauptfliessrichtung 1 gebildet wird. Aufgrund der herrschenden Druckverhältnisse bei Betrieb des erfindungsgemässen Leitungssystems 100 wird der erste zuströmseitige Leitungsabschnitt 40 und der erste abströmseitige Leitungsabschnitt 50 der Nebenleitung in Hauptfliessrichtung 1 durchströmt, während in Gegenfliessrichtung 2 der zweite zuströmseitige Leitungsabschnitt 41 und der zweite abströmseitige Leitungsabschnitt 51 der Nebenleitung durchströmt wird. In der in Fig. 5 gezeigten Ausführungsform sind der Bereich 60 der Hauptleitung mit vergrössertem Innendurchmesser und der Bereich 70 der Hauptleitung mit verkleinertem Innendurchmesser beabstandet zueinander angeordnet. Sie können jedoch wie schon in den Figuren 1-4 gezeigt auch benachbart zueinander angeordnet sein.

### Bezugszeichenliste

- 100: Leitungssystem
- 1: Hauptfliessrichtung der Hauptleitung
- 2: Gegenfliessrichtung der Hauptleitung
- 10: Hauptleitung
- 20, 21, 22: Nebenleitung
- 30, 31, 32: Entnahmestelle
- 40, 41, 42: zuströmseitigen Leitungsabschnitt der Nebenleitung
- 50, 51, 52: abströmseitigen Leitungsabschnitt der Nebenleitung
- 60, 61, 62: Bereich der Hauptleitung mit vergrössertem Innendurchmesser (Rohrerweiterung der Hauptleitung)
- 70, 71, 72: Bereich der Hauptleitung mit verkleinertem Innendurchmesser (Rohrverengung der Hauptleitung)
- 80, 80': Erweiterungsabschnitte
- 90, 90': Verengungsabschnitte
- α: Winkel der Nebenleitung zur Längsachse der Hauptleitung

## Patentansprüche

1. Leitungssystem (100), insbesondere ein Frisch- oder Reinwasser-Rohrleitungssystem, mit
a) einer Hauptleitung (10), die von einem Medium durchströmbar ist, und mit
b) zumindest einer davon abgezweigten, einen Bypass bildenden Nebenleitung (20), an die wenigstens eine Entnahmestelle (30) anschliessbar ist,
- wobei die Hauptleitung (10) mindestens einen Bereich (60) mit vergrössertem Innendurchmesser und mindestens einen Bereich (70) mit verkleinertem Innendurchmesser aufweist,
- wobei jeweils der zuströmseitige Leitungsabschnitt (40, 41) einer Nebenleitung einem Bereich (60) der Hauptleitung mit vergrössertem Innendurchmesser zugeordnet ist,
- wobei jeweils der abströmseitige Leitungsabschnitt (50, 51) einer Nebenleitung einem Bereich der Hauptleitung (70) mit verkleinertem Innendurchmesser zugeordnet ist,
**dadurch gekennzeichnet, dass**
zumindest einer der zuströmseitigen oder abströmseitigen Leitungsabschnitte (40, 41, 50, 51) der Nebenleitung(en) mit der Hauptleitung (10) in einem schiefen Winkel α zur Längsachse der Hauptleitung in Hauptfliessrichtung (1) verbunden ist.

2. Leitungssystem nach Anspruch 1, **dadurch gekennzeichnet, dass** der einer Nebenleitung (20) zugeordnete Bereich (70) der Hauptleitung mit verkleinertem Innendurchmesser dem der gleichen Nebenleitung zugeordneten Bereich (60) der Hauptleitung mit vergrössertem Innendurchmesser in Hauptfliessrichtung (1) der Hauptleitung vorgeschaltet ist.

3. Leitungssystem nach Anspruch 1 oder Anspruch 2, **dadurch gekennzeichnet, dass** der zuströmseitige Leitungsabschnitt (40) der Nebenleitung mit der Hauptleitung (10) in einem stumpfen Winkel α, vorteilhafterweise in einem Winkel von mindestens 135°, zur Längsachse der Hauptleitung in Hauptfliessrichtung (1) verbunden ist.

4. Leitungssystem nach einem der Ansprüche 1 bis 3, **dadurch gekennzeichnet, dass** der abströmseitige Leitungsabschnitt (50) der Nebenleitung mit der Hauptleitung (10) in einem spitzen Winkel α zur Längsachse der Hauptleitung in Hauptfliessrichtung (1) verbunden ist.

5. Leitungssystem nach einem der Ansprüche 1 bis 4, **dadurch gekennzeichnet, dass** die Nebenleitung (20) einen ersten zuströmseitigen Leitungsabschnitt (40) und einen zweiten zuströmseitigen Leitungsabschnitt (41) aufweist, und sowohl der erste zuströmseitige Leitungsabschnitt (40) als auch der zweite zuströmseitige Leitungsabschnitt (41) der Nebenleitung demselben Bereich (60) der Hauptleitung mit vergrössertem Innendurchmesser zugeordnet ist.

6. Leitungssystem nach Anspruch 5, **dadurch gekennzeichnet, dass** der zweite zuströmseitige Leitungsabschnitt (41) der Nebenleitung mit der Hauptleitung (10) in einem spitzen Winkel α zur Längsachse der Hauptleitung in Hauptfliessrichtung (1) verbunden ist.

7. Leitungssystem nach einem der Ansprüche 1 bis 6, **dadurch gekennzeichnet, dass** die Nebenleitung (20) einen ersten abströmseitigen Leitungsabschnitt (50) und einen zweiten abströmseitigen Leitungsabschnitt (51) aufweist, und sowohl der erste abströmseitige Leitungsabschnitt (50) als auch der zweite abströmseitige Leitungsabschnitt (51) der Nebenleitung demselben Bereich (70) der Hauptleitung mit verkleinertem Innendurchmesser zugeordnet ist.

8. Leitungssystem nach Anspruch 7, **dadurch gekennzeichnet, dass** der zweite abströmseitige Leitungsabschnitt (51) der Nebenleitung mit der Hauptleitung (10) in einem spitzen Winkel α, vorteilhafterweise in einem Winkel von maximal 45°, zur Längsachse der Hauptleitung in Hauptfliessrichtung (1) verbunden ist.

9. Leitungssystem nach einem der Ansprüche 1-8, **dadurch gekennzeichnet, dass** alle zuströmseitigen Leitungsabschnitte (40, 41) und alle abströmseitigen Leitungsabschnitte (50, 51) aller Nebenleitungen (20) mit der Hauptleitung (10) in einem schiefen Winkel α zur Längsachse der Hauptleitung in Hauptfliessrichtung (1) verbunden sind.

10. Leitungssystem nach einem der Ansprüche 1-9, **dadurch gekennzeichnet, dass** der Bereich (60) der Hauptleitung mit vergrössertem Innendurchmesser zwei, vorzugsweise etwa gleich grosse, Erweiterungsabschnitte (80, 80') hat, die jeweils trichter- oder kegelstumpfförmig ausgebildet sind und sich zu ihren einander abgewandten Enden hin verjüngen.

11. Leitungssystem nach einem der Ansprüche 1-10, **dadurch gekennzeichnet, dass** der Bereich (70) der Hauptleitung mit verkleinertem Innendurchmesser zwei, vorzugsweise etwa gleich grosse, Verengungsabschnitte (90, 90') hat, die jeweils trichter- oder kegelstumpfförmig ausgebildet sind und sich zu ihren einander zugewandten Enden hin verjüngen.
